# FASCICULE DE BREVET EUROPEEN

(11) **EP 1 935 396 B1**
(45) Date de publication et mention de la délivrance du brevet: **18.01.2012**
(21) Numéro de dépôt: 07301719.6
(22) Date de dépôt: 20.12.2007
(51) Int. Cl.: A61K 8/22, A61K 8/44, A61Q 5/04, A61K 8/46, A61K 8/55, A61K 8/87

(54) **Procédé de déformation permanente des fibres kératiniques comprenant une étape d'application d'une composition réductrice faiblement concentrée et une étape intermédiaire de séchage**
Verfahren zur dauerhaften Verformung von Keratinfasern, das eine Anwendungsphase einer gering konzentrierten reduzierenden Zusammensetzung und eine Zwischentrockenphase umfasst
Method for permanently deforming keratinous fibres including a step of applying a reducing composition with a low concentration and an intermediate drying step

(30) Priorité: 22.12.2006 FR 0655931
(43) Date de publication de la demande: 25.06.2008
(73) Titulaire: L'Oréal, 75008 Paris (FR)
(72) Inventeur: CAMPAIN, Catherine, 75019, PARIS (FR); DUTHEIL-GOURET, Katia, 78370, PLAISIR (FR); PETIT, Gaëlle, 75018, PARIS (FR); GEVAUDAN, Françoise, 95250, BEAUCHAMP (FR)
(74) Mandataire: Catherine, Alain

(56) Documents cités:
- EP-A- 1 247 518
- DE-A1- 3 138 813
- DE-A1- 19 709 437
- US-A- 5 988 180
- US-A- 6 116 250

## Description

La présente invention se rapporte à un procédé de déformation permanente des cheveux comprenant notamment une étape de séchage des cheveux entre une étape d'application d'une composition réductrice faiblement concentrée, et une étape d'application d'une composition oxydante.

Il existe aujourd'hui de nombreux produits permettant de se coiffer facilement et d'apporter de la texture et de la masse à des cheveux, et notamment à des cheveux fins. On citera par exemple les mousses et les gels de coiffage ou les laques. Ces produits permettent de mettre en forme la chevelure mais s'éliminent au shampooing et nécessitent une application quotidienne.

Pour obtenir une déformation durable des cheveux, la technique la plus usuelle consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, généralement à l'eau, à reconstituer dans un second temps lesdites liaisons disulfures en appliquant, sur les cheveux préalablement mis sous tension par exemple par des bigoudis, une composition oxydante (étape d'oxydation, dite aussi de fixation) de façon à donner finalement aux cheveux la forme recherchée. Ces étapes sont effectuées généralement en maintenant les fibres kératiniques sous une contrainte mécanique, par exemple au moyen de bigoudis.

La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, mettant en oeuvre des mousses, des gels de coiffage ou des laques.

Néanmoins, l'enroulage des bigoudis est un geste technique, difficilement maîtrisé par les consommateurs non avertis. Des techniques visant à supprimer l'étape de mise sous tension des cheveux par des bigoudis, pour obtenir une maîtrise de la chevelure ont donc été développées.

Toutefois, du fait de l'absence de bigoudis ces procédés n'apportent pas une tension forte aux cheveux, et la mise en forme des cheveux ainsi obtenue avec ces procédés est à la fois peu intense et de faible durée.

Un autre inconvénient des techniques de déformation permanente usuelles décrites ci-dessus, qui nécessitent généralement l'utilisation d'acide thioglycolique ou un de ses sels à de fortes concentrations, réside dans la dégradation de la couleur des cheveux colorés artificiellement.

Ainsi, par exemple, si les techniques de déformation permanente des cheveux décrites précédemment sont appliquées sur des cheveux ayant fait l'objet au préalable d'une coloration artificielle, elles entraînent une dégradation ou un décapage de cette coloration artificielle.

Aucun des procédés décrits ne conduit à un procédé de déformation permanente totalement satisfaisant vis-à-vis des problèmes mentionnés ci-dessus.

Il existe donc un besoin pour un procédé de déformation permanente des cheveux qui ne dégrade pas la coloration artificielle des cheveux, et permette de réaliser, de manière durable, une augmentation du volume des cheveux à la fois au niveau de la racine par décollement de ceux-ci, mais également sur toute leur longueur. Ce procédé devra en outre être particulièrement efficace sur les cheveux courts et fins et apporter une meilleure coiffabilité, avec un matériel facile à poser.

Ce besoin est très présent dans le domaine des produits destinés au grand public.

La demanderesse a découvert que les buts ci-dessus sont atteints par un procédé de déformation permanente des fibres kératiniques comprenant notamment une étape de séchage des fibres kératiniques entre une étape d'application d'une composition réductrice, de faible concentration et une étape de fixation par oxydation.

L'invention a donc pour objet un procédé de déformation permanente des fibres kératiniques, en particulier des cheveux, comprenant :
- une étape de mise sous tension des fibres kératiniques,
- une étape d'application sur les fibres kératiniques d'une composition comprenant un polymère fixant,
- une étape d'application sur les fibres kératiniques d'une composition réductrice, pour réduire les liaisons disulfure de la kératine, ladite composition réductrice comprenant dans un milieu cosmétiquement acceptable, de 0,1 à 3% en poids d'au moins un agent réducteur par rapport au poids total de la composition réductrice, puis après un éventuel rinçage,
- une étape de séchage des fibres kératiniques, puis
- une étape de fixation par oxydation, pour reformer lesdites liaisons, par application sur les fibres kératiniques d'une composition oxydante,
- une étape d'enlèvement des matériels de mise sous tension

L'application sur les fibres kératiniques d'une composition réductrice faiblement concentrée garantit un bon respect des fibres kératiniques et en particulier des cheveux colorés. En particulier, un effet protecteur de la couleur artificielle des cheveux est observé tant lors de la mise en oeuvre du procédé objet de l'invention, qu'ultérieurement après plusieurs lavages par shampoings. En d'autres termes, le procédé de déformation permanente des fibres kératiniques objet de l'invention ne dégrade pas la coloration artificielle des cheveux.

L'application sur les fibres kératiniques d'une composition réductrice faiblement concentrée permet également de mettre en oeuvre le procédé objet de l'invention plusieurs fois, de manière rapprochée dans le temps sur les mêmes fibres kératiniques, sans les endommager, ce qui n'est pas possible avec des procédés mettant en oeuvre des réducteurs plus concentrés.

L'application sur les fibres kératiniques d'une composition réductrice faiblement concentrée permet aussi une diminution des odeurs dégagées par rapport aux procédés de mise en forme classiques.

L'application sur les fibres kératiniques d'une composition réductrice faiblement concentrée permet enfin de faire varier le temps de pause de cette composition sur les cheveux en fonction de leur nature, et n'oblige pas à changer de type de composition réductrice en fonction du type de cheveux à traiter. Ce dernier point représente un avantage important pour le grand public, puisqu'il n'est pas nécessaire de faire un choix entre plusieurs types de compositions réductrices en fonction de la nature des cheveux mais seulement de faire varier le temps de pause de celui-ci.

L'étape de mise sous tension des fibres kératiniques peut être réalisée par tout moyen et par exemple avec des élastiques, des pinces, des peignes, des barrettes, des boudins ou encore avec des bigoudis de type « tulipe ».

Des bigoudis tulipe sont préférés, et se composent d'une tige allongée formant un corps terminé à une extrémité par une tête dotée d'au moins une perforation. Le corps du bigoudi est réalisé en matière souple et flexible, de sorte que son extrémité libre puisse être introduite dans la perforation de la tête et y être maintenue par serrage élastique. Un bigoudi du type défini ci-dessus, convenant particulièrement à la mise en oeuvre du procédé objet de l'invention est par exemple décrit dans la demande de brevet FR2602650.

L'utilisation d'un bigoudi « tulipe» permet de contrôler la tension appliquée aux cheveux et permet ainsi de donner une forme arrondie aux mèches de cheveux pendant la durée du traitement, sans traction excessive contrairement à ce que l'on observe avec la plupart des bigoudis cylindriques. Les bigoudis « tulipe » présentent aussi l'avantage d'être faciles à poser et d'être plus confortables pour le cuir chevelu que les bigoudis cylindriques traditionnels. Selon la forme de coiffure et la quantité de boucles désirée, l'enroulage se fait avec des mèches plus ou moins épaisses.

Le procédé défini ci-dessus comprend une étape de séchage des fibres kératiniques. Le séchage des fibres kératiniques peut être un séchage partiel ou un séchage complet, et peut être réalisée par exemple avec un sèche-cheveux domestique ou encore un casque ou un bonnet chauffant, ou encore être effectuée grâce à un essorage soigneux. De préférence, le séchage des fibres kératiniques est un séchage complet. De préférence, on utilise un système chauffant pour sécher les cheveux.

La composition réductrice utilisée dans le procédé selon l'invention comprend généralement, dans un milieu cosmétiquement acceptable, au moins un agent réducteur choisi parmi les sulfites, les bisulfites, les thiols et les phosphines.

A titre de sulfites et de bisulfites utilisables, on peut citer les sulfites ou bisulfites des métaux alcalins ou alcalino-terreux ou d'ammonium et en particulier le sulfite ou le bisulfite de sodium, de potassium ou de monoéthanolamine.

De préférence, le ou les thiols utilisés comme agents réducteurs dans la composition réductrice sont choisis parmi la cystéine et ses dérivés, comme la N-acétylcystéïne, la cystéamine et ses dérivés, comme ses dérivés acylés en C1-C4 tels que la N-acétyl cystéamine et la N-propionyl cystéamine, l'acide thiolactique et ses esters, comme le monothiolactate de glycérol, l'acide thioglycolique et ses esters, comme le monothioglycolate de glycérol ou de glycol, et le thioglycérol.

Comme thiols utilisables dans la composition réductrice utilisée selon l'invention, on peut encore citer les N-mercapto-alkylamides de sucres tels que le N-(mercapto-2-éthyl)gluconamide, l'acide P-mercaptopropionique et ses dérivés, l'acide thiomalique, la panthétéïne, les N-(mercaptoalkyl)w-hydroxyalkylamides tels que ceux décrits dans la demande de brevet EP-A-354 835 et les N-mono- ou N,N-dialkylmercapto 4-butyramides tels que ceux décrits dans la demande de brevet EP-A-368 763, les aminomercaptoalkylamides tels que ceux décrits dans la demande de brevet EP-A-432 000 et les alkylaminomercaptoalkylamides tels que ceux décrits dans la demande de brevet EP-A-514 282, le mélange de thioglycolate d'hydroxy-2 propyle (2/3) et de thioglycolate d'hydroxy-2 méthyl-1 éthyle (67/33) décrit dans la demande de brevet FR-A-2 679 448.

La demanderesse a montré que l'application d'une composition réductrice comprenant de la cystéine, permet d'obtenir une augmentation du volume des cheveux à la fois au niveau de la racine par décollement de ceux-ci, mais également sur toute leur longueur, y compris sur des cheveux fins et courts.

De manière tout a fait préférée la composition réductrice est choisie parmi la L-cystéine, la D-cystéine, la L,D-cystéine, et leurs sels, l'acide thiolactique ses sels et ses esters, l'acide thioglycolique ses sels et ses esters, et leurs mélanges.

Encore plus préférentiellement, le réducteur est la cystéine.

Le ou les agents réducteurs représentent de préférence de 0,3 à 3%, en poids par rapport au poids total de la composition réductrice.

Selon un mode de réalisation préféré, on laisse la composition réductrice agir pendant 1 à 50 minutes, de préférence pendant 1 à 30 minutes. Le temps de pause de la composition réductrice sera modifié en fonction de la nature des cheveux.

Pendant ou après l'application de la composition réductrice, les fibres kératiniques peuvent être soumises à un traitement thermique par chauffage, par exemple à une température comprise entre 30 et 250°C pendant tout ou partie du temps de pose défini ci-dessus. Dans la pratique, cette opération peut être conduite au moyen d'un casque de coiffure, d'un sèche-cheveux, d'un fer rond ou d'un fer plat, d'un dispensateur de rayons infrarouges et d'autres appareils chauffants, et dans certains cas sous film plastique.

Le pH de la composition réductrice selon l'invention est de préférence compris entre 7,5 et 11, de préférence entre 8 et 9,5.

Le pH de la composition réductrice selon l'invention peut être obtenu et/ou ajusté classiquement par ajout soit d'agents alcalins, tels que par exemple l'ammoniaque, la monoéthanolamine, la diéthanolamine, la triéthanolamine, l'isopropanolamine, le 2-méthyl-2-amino-1-propanol, la propanediamine-1,3, un carbonate ou bicarbonate alcalin ou d'ammonium, le carbonate et le bicarbonate de sodium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique, l'acide borique, l'acide citrique et l'acide phosphorique.

Dans le but d'améliorer les propriétés cosmétiques des fibres capillaires ou encore d'atténuer ou d'éviter leur dégradation, la composition réductrice utilisée dans le procédé selon l'invention peut également comprendre un ou plusieurs actifs cosmétiques.

Ce ou ces actifs sont généralement choisis parmi les silicones volatiles ou non, linéaires ou cycliques, aminées ou non, les polymères cationiques, anioniques, non ioniques ou amphotères, les peptides et leurs dérivés, les hydrolysats de protéines, les cires naturelles ou synthétiques, et en particulier les alcools gras, les agents de gonflement et de pénétration ou permettant de renforcer l'efficacité du ou des réducteurs, ainsi que d'autres composés actifs tels que les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents chélatants, les agents opacifiants, les colorants, les filtres solaires, les charges, les vitamines ou provitamines, les huiles minérales, végétales ou synthétiques, ainsi que les parfums, les conservateurs, les stabilisants, et leurs mélanges.

De préférence, la composition réductrice comprend un polymère cationique et/ou au moins une silicone.

Au sens de la présente demande, "polymère cationique" désigne tout polymère contenant des groupements cationiques ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n ° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (I), (II), (III) ou (IV) suivantes: 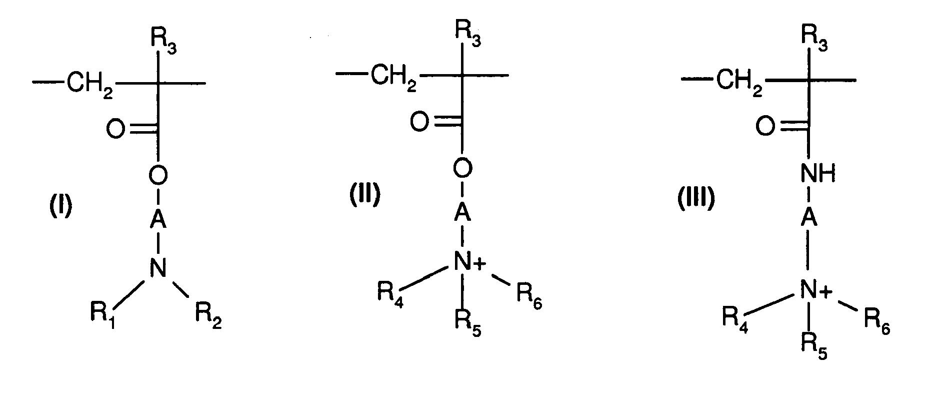 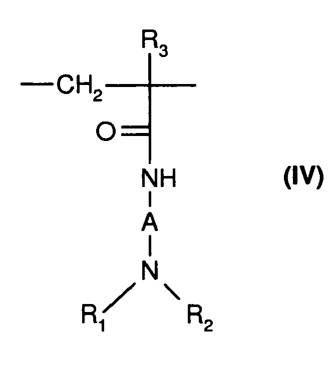 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyl-triméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxy-éthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthyl-aminopropyle quaternisés tel que le produit vendu sous la dénomination "GAFQUAT HS 100" par la société ISP .
(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 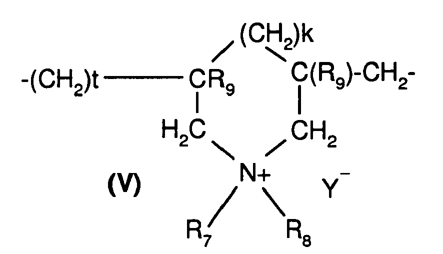 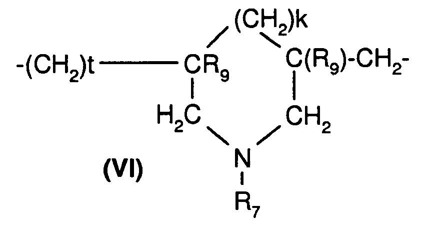 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(10) Le polymère de diammonium quaternaire contenant des motifs récurrents répondant à la formule : 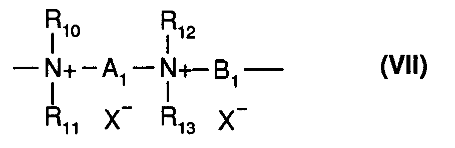 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀,
   R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁,
   R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :
         - (CH₂-CH₂-O)x-CH₂-CH₂-
         - [CH₂-CH(CH₃)-O]y-CH₂-CH(CH₃)-
         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent -CH₂-CH₂-S-S-CH₂-CH₂-;
      d) un groupement uréylène de formule : -NH-CO-NH-.
   De préférence, X- est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 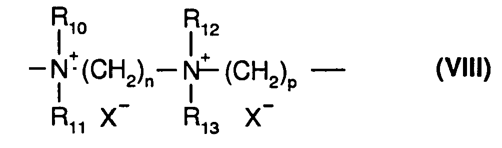 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique. On peut citer en particulier le chlorure d'hexadiméthrine (nom INCI), commercialisé par la société CHIMEX sous la référence MEXOMERE PO ;
(11) Les polyammoniums quaternaires constitués de motifs récurrents de formule (IX) : 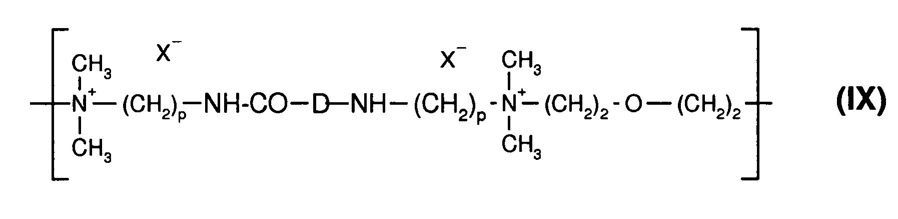 dans laquelle p désigne un nombre entier variant de 1 à 6 environ, D peut être nul ou peut représenter un groupement -(CH₂)ᵣ -CO- dans lequel r désigne un nombre égal à 4 ou à 7, X- est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de " POLYETHYLENEGLYCOL (15) TALLOW POLYAMINE "dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre un polymère cationique de la famille (10) et par exemple le chlorure d'hexadiméthrine (nom INCI), commercialisé par la société CHIMEX sous la référence MEXOMERE PO.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

Comme expliqué précédemment, le ou les actifs cosmétiques pouvant être utilisés dans la composition selon l'invention peuvent être également choisis parmi les silicones.

Les silicones éventuellement présentes dans la composition réductrice selon l'invenion sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press.

Les silicones peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5.
   Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/ méthylalkylsiloxane, tel que la "SILICONE VOLATILE FZ 3109" commercialisée par la société UNION CARBIDE, de structure chimique (X): 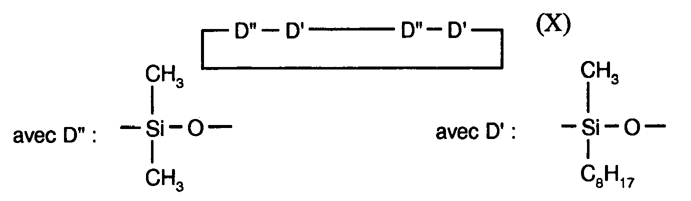 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. II s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC .

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles SILBIONE de la série 70 641 de RHONE POULENC;
. les huiles des séries RHODORSIL 70 633 et 763 de RHONE POULENC ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone pouvant être présentes dans la composition réductrice selon l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane,
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables sont les mélanges suivants:
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes éventuellement présentes dans la composition oxydante du procédé selon l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées éventuellement présentes dans la composition réductrice selon l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE, les produits 176-12096G de GENERAL ELECTRIC, les produits KF-860, 861 et 864 de SHINETSU ou les produits commercialisés sous les dénominations Q2 8220 ou DCZ-8566 et DOW CORNING 929 ou 939 ou DCZ-8299 ou QZ7224 par la société DOW CORNING. Les groupements aminés substitués sont en particulier des groupements aminoalkyle en C₁-C₄ ; on peut citer les silicones aminées comportant des groupements alcoxy telle que la silicone BELSIL ADM LOG 1 commercialisé par la société WACKER
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 ;
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255";
- des groupements hydroxyacylamino, comme les polyorganosiloxanes décrits dans la demande EP 342 834. On peut citer par exemple le produit Q2-8413 de la société DOW CORNING.

De préférence, la silicone est une silicone aminée.

Comme expliqué précédemment, le procédé selon l'invention comprend une étape d'application d'une composition oxydante.

La composition oxydante comprend généralement au moins un agent oxydant choisi parmi le peroxyde d'hydrogène, le peroxyde d'urée, les bromates alcalins, les polythionates, les persels, tels que les perborates, les percarbonates et les persulfates.

De préférence, l'agent oxydant est le peroxyde d'hydrogène.

Le ou les agents oxydants représentent généralement de 0,1 à 10%, de préférence de 0,5 à 5%, en poids par rapport au poids total de la composition oxydante.

De préférence, lorsque l'agent oxydant est du peroxyde d'hydrogène en solution aqueuse, la composition oxydante utilisée dans le procédé selon l'invention contient au moins un agent stabilisant de l'eau oxygénée.

On peut citer particulier les pyrophosphates des métaux alcalins ou alcalino-terreux, tel que le pyrophosphate de tétrasodium, les stannates des métaux alcalins ou alcalino-terreux, la phénacétine ou les sels d'acides et d'oxyquinoléine, comme le sulfate d'oxyquinoléine. De manière plus avantageuse encore, on utilise au moins un stannate en association ou non à au moins un pyrophosphate.

Le ou les agents stabilisants de l'eau oxygénée représentent généralement de 0,0001 % à 5% en poids et de préférence de 0,01 à 2% en poids par rapport au poids total de la composition oxydante.

Dans le but d'améliorer les propriétés cosmétiques des fibres capillaires ou encore d'atténuer ou d'éviter leur dégradation, la composition oxydante utilisée dans le procédé selon l'invention peut également comprendre un ou plusieurs actifs cosmétiques, tels que ceux mentionnés précédemment au sujet de la composition réductrice.

Généralement, le pH de la composition oxydante varie de 1,5 à 4,5, de préférence de 2 à 3,5.

De préférence, on laisse la composition oxydante agir pendant 2 à 30 minutes, de préférence pendant 2 à 15 minutes, mieux de 2 à 7 minutes.

De préférence, le procédé objet de l'invention comprend une étape d'application d'une composition de soin contenant un polymère cationique.

Tous les polymères cationiques décrits ci-dessus en ce qui concerne la composition réductrice peuvent être utilisés dans la composition de soin.

Parmi tous les polymères cationiques susceptibles d'être utilisés dans le cadre de la présente invention, on préfère mettre en oeuvre dans la composition réductrice les cyclopolymères, en particulier les homolymères du chlorure de diméthyldiallylammonium commercialisé sous la dénomination « MERQUAT® 100 » par la Société MERCK, les polymères de diammonium quaternaire de formule (VIII) ou de formule (IX) et en particulier le MEXOMERE PO.

La silicone préférée est la silicone WACKER BELSIL ADM LOG 1.

Une étape d'application d'une composition de soin permet de limiter ou d'éviter une sensibilisation des cheveux qui pourrait résulter du traitement des cheveux par des agents réducteurs et des agents oxydants au cours du procédé de déformation permanente objet de l'invention. La composition de soin telle que définie ci-dessus permet également de protéger la couleur artificielle des cheveux.

La composition oxydante et la composition de soin utilisées dans le procédé selon l'invention peuvent également comprendre un ou plusieurs actifs cosmétiques, tels que ceux mentionnés précédemment au sujet de la composition réductrice.

Le véhicule des compositions réductrice, oxydante et de soin utilisées dans le procédé selon l'invention est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants organiques peuvent alors être présents dans des concentrations comprises entre environ 0,1 et 20% et, de préférence, entre environ 1 et 10% en poids par rapport au poids total de la composition.

Les pH de la composition réductrice, de la composition oxydante et de la composition de soin utilisées dans le procédé selon l'invention peuvent être obtenus et/ou ajustés classiquement par ajout soit d'agents alcalins, tels que par exemple l'ammoniaque, la monoéthanolamine, la diéthanolamine, la triéthanolamine, l'isopropanolamine, le 2-méthyl-2-amino-1-propanol, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique, l'acide borique, l'acide citrique et l'acide phosphorique.

La composition réductrice, la composition oxydante et la composition de soin utilisées dans le procédé selon l'invention peuvent se présenter indépendamment l'une de l'autre sous forme de lotion, de gel épaissi ou non, de mousse, ou de crème.

Le procédé objet de l'invention comprend une étape d'application d'une composition comprenant un polymère fixant, entre l'étape de mise sous tension mécanique des fibres kératiniques et l'étape d'application d'une composition réductrice.

Pour la mise en oeuvre de la composition définie immédiatement ci-dessus, on peut, en principe, utiliser n'importe quel polymère fixant naturel ou synthétique permettant de conférer une forme à la chevelure ou de modifier la forme de ladite chevelure. De préférence le polymère fixant utilisé est anionique ou amphotère ; il peut aussi s'agir de mélanges de polymères anioniques et amphotères.

Les polymères anioniques généralement utilisés, sont des polymères comportant des groupes dérivés d'acides carboxylique, sulfonique ou phosphorique, et présentant une masse moléculaire en poids comprise entre 500 et 5 000 000.

Les groupements carboxyliques sont apportés par des monomères monoacides ou diacides carboxyliques insaturés tels que ceux répondant à la formule (XI) : 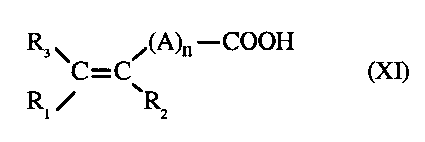 dans laquelle n est un nombre entier de 0 à 10, A désigne un groupement méthylène, éventuellement relié à l'atome de carbone du groupement insaturé ou au groupement méthylène voisin lorsque n est supérieur à 1, par l'intermédiaire d'un hétéroatome tel que l'oxygène ou le soufre, R1 désigne un atome d'hydrogène, un groupement phényle ou benzyle, R2 désigne un atome d'hydrogène, un groupement alkyle inférieur ou carboxyle, R3 désigne un atome d'hydrogène, un groupement alkyle inférieur, un groupement -CH2-COOH, phényle ou benzyle.

Dans la formule (XI) ci-dessus, un groupement alkyle inférieur comporte de préférence de 1 à 4 atomes de carbone et désigne en particulier, les groupements méthyle et éthyle.

Les polymères anioniques à groupements carboxyliques ou sulfoniques préférés sont :
A) les homo- ou copolymères d'acide acrylique ou méthacrylique ou leurs sels, dont les copolymères d'acide acrylique et d'acrylamide et les copolymères acide méthacrylique/acide acrylique/acrylate d'éthyle/méthacrylate de méthyle, en particulier l'AMERHOLD DR 25 commercialisé par la société AMERCHOL, et les sels de sodium des acides polyhydroxycarboxyliques. On peut citer également les copolymères acide méthacrylique/acrylate d'éthyle, notamment en dispersion aqueuse, tels que les LUVIFLEX SOFT et LUVIMER MAE commercialisés par la société BASF.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371. On peut également citer les copolymères d'acide acrylique et de méthacrylate d'alkyle en C1-C4.
C) les copolymères dérivés d'acide crotonique tels que ceux comportant dans leur chaîne des motifs acétate ou propionate de vinyle, et éventuellement d'autres monomères tels que les ester allylique ou méthallylique, éther vinylique ou ester vinylique d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β-cyclique. De tels polymères sont décrits entre autres dans les brevets français numéros 1 222 944, 1 580 545, 2 265 782, 2 265 781, 1 564 110 et 2 439 798. Des produits commerciaux entrant dans cette classe sont les résines 28-29-30, 26-13-14 et 28-13-10 vendues par la société NATIONAL STARCH.
   On peut citer aussi comme copolymère dérivé d'acide crotonique les terpolymères acide crotonique/acétate de vinyle/ t.butylbenzoate de vinyle et en particulier le MEXOMERE PW fourni par la société CHIMEX.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2 047 398, 2 723 248, 2 102 113, le brevet GB 839 805, et notamment ceux vendus sous les dénominations GANTREZ® AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français 2 350 384 et 2 357 241 de la demanderesse.
E) Les polyacrylamides comportant des groupements carboxylates.
F) Les polymères comprenant les groupements sulfoniques. Ces polymères peuvent être des polymères comportant des motifs vinylsulfonique, styrène-sulfonique, naphtalène-sulfonique, acrylamido-alkylsulfonique, sulfoisophtalates.
   Ces polymères peuvent être notamment choisis parmi :
   - les sels de l'acide polyvinylsulfonique ayant une masse moléculaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinylpyrrolidone ;
   - les sels de l'acide polystyrène-sulfonique, les sels de sodium, ayant une masse moléculaire d'environ 500 000 et d'environ 100 000. Ces composés sont décrits dans le brevet FR 2198719 ;
   - les sels d'acides polyacrylamide-sulfoniques tels que ceux mentionnés dans le brevet US 4 128 631 ;
G) les polymères siliconés anioniques greffés ;
   Les polymères siliconés greffés utilisés sont choisis préférentiellement parmi les polymères à squelette organique non-siliconé greffé par des monomères contenant un polysiloxane, les polymères à squelette polysiloxanique greffé par des monomères organiques non-siliconés et leurs mélanges.
   Dans tout ce qui suit ou qui précède, on entend désigner par silicone ou polysiloxane, en conformité avec l'acceptation générale, tout polymère oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C1-C10 et en particulier méthyle, les radicaux fluoralkyles, les radicaux aryles et en particulier phényle, et les radicaux alcényles et en particulier vinyle ; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyles ou hydroxyalkyles, les groupements aminés substitués ou non, les groupements amides, les radicaux acyloxy ou acyloxyalkyles, les radicaux hydroxyalkylamino ou aminoalkyles, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites « organomodifiées »).
   Dans ce qui suit, on entend désigner par « macromère polysiloxane » en conformité avec l'acceptation générale, tout monomère contenant dans sa structure une chaîne polymère du type polysiloxane.
   Les polymères à squelette organique non-siliconé greffé par des monomères contenant un polysiloxane utilisé selon la présente invention sont constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane.
   Les monomères organiques non-siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.
   Les polymères à squelette organique non-siliconé greffé par des monomères contenant un polysiloxane utilisables peuvent être obtenus selon tout moyen connu de l'homme de l'art, en particulier par réaction entre (i) un macromère polysiloxane de départ correctement fonctionnalisé sur la chaîne polysiloxanique et (ii) un ou plusieurs composés organiques non-siliconés, eux-mêmes correctement fonctionnalisés par une fonction qui est capable de venir réagir avec le ou les groupements fonctionnels portés par ladite silicone en formant une liaison covalente ; un exemple classique d'une telle réaction est la réaction radicalaire entre un groupement vinylique porté sur une des extrémités de la silicone avec une double liaison d'un monomère à insaturation éthylénique de la chaîne principale.
   Les polymères à squelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0412704, EP-A-0412707, EP-A-0640105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.
   Une famille particulière de polymères siliconés greffés convenant pour la réalisation de la présente invention est constituée par les copolymères greffés siliconés comprenant :
   a) de 0 à 98 % en poids d'au moins un monomère (A') lipophile de faible polarité lipophile à insaturation éthylénique, polymérisable par voie radicalaire ;
   b) de 1 à 98 % en poids d'au moins un monomère (B') hydrophile polaire à insaturation éthylénique, copolymérisable avec le ou les monomères du type (A) ;
   c) de 0,01 à 50 % en poids d'au moins un macromère polysiloxane (C') de formule générale (XII) :

      X(Y)nSi(R)3-mZm (XII)

      Où :
      X désigne un groupe vinylique copolymérisable avec les monomères (A') et (B');
      Y désigne un groupe de liaison divalent ;
      R désigne un hydrogène, un alkyle ou un alcoxy en C1-C6, un aryle C6-C12 ;
      Z désigne un motif polysiloxane monovalent ayant un poids moléculaire moyen en nombre d'au moins 500 ; n est 0 ou 1 et m est un entier allant de 1 à 3 ; les pourcentages étant calculés par rapport au poids total des monomères (A'), (B') et (C').
   Ces polymères sont décrits ainsi que leurs procédés de préparation dans les brevets US 4,963,935, US 4,728,571 et US 4,972,037 et les demandes de brevet EP-A-0412704, EP-A-0412707, EP-A-0640105. Ils ont un poids moléculaire moyen en nombre de préférence allant de 10 000 à 2 000 000 et de préférence une température de transition vitreuse Tg ou une température de fusion cristalline Tm d'au moins -20°C.
   On peut citer comme exemples de monomères lipophiles (A'), les esters d'acide acrylique ou méthacrylique d'alcools en C1-C18 ; le styrène ; les macromères polystyrène ; l'acétate de vinyle ; le propionate de vinyle ; l'alpha-méthylstyrène ; le tertio-butylstyrène ; le butadiène ; le cyclohexadiène ; l'éthylène ; le propylène ; le vinyltoluène ; les esters d'acide acrylique ou méthacrylique et de 1,1-dihydroperfluoroalcanol ou de ses homologues ; les esters d'acide acrylique ou méthacrylique et de oméga-hydrydofluoroalcanol ; les esters d'acide acrylique ou méthacrylique et de fluoroalkylsulfoamido-alcool ; les esters d'acide acrylique ou méthacrylique et d'alcool fluoroalkylique ; les esters d'acide acrylique ou méthacrylique et de fluoréther d'alcool ; ou leurs mélanges.
   Les monomères (A') préférentiels sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-méthyl perfluoroctane sulfoamido)éthylacrylate ; le 2-(N-butylperflurooctane sulfoamido)éthylacrylate et leurs mélanges.
   On peut citer comme exemples de monomères polaires (B'), l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butylacrylamide, l'acide maléique, l'anhydride maléique et leurs demi-esters, les (méth)acrylates hydroxyalkylés, le chlorure de diallyldiméthylammonium, la vinylpyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinylcaprolactame ou leurs mélanges. Les monomères (B') préférentiels sont choisis dans le groupe constitué par l'acide acrylique, le N,N-diméthylacrylamide, le méthacrylate de diméthylaminoéthyle, le méthacrylate de diméthylaminoéthyle quaternisé, la vinylpyrrolidone et leurs mélanges.
   A titre de monomères polaires (B'), les polymères siliconés greffés anioniques utilisés selon l'invention contiennent au moins un monomère anionique.
   Les macromères polysiloxane (C') de formule (XII) préférentiels sont choisis parmi ceux répondant à la formule générale suivante (XIII) : 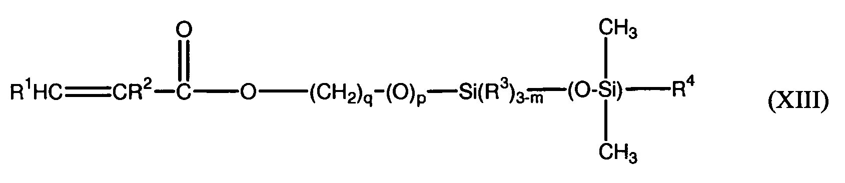 dans laquelle :
   R1 est l'hydrogène ou -COOH (de préférence hydrogène) ;
   R2 est l'hydrogène, méthyle ou -CH2COOH (de préférence méthyle) ;
   R3 est un groupe alkyle, alcoxy ou alkylamino en C1-C6, aryle en C1-C12 ou hydroxyle (de préférence méthyle) ;
   R4 est un groupe alkyle, alcoxy ou alkylamino en C1-C6, aryle en C1-C12 ou hydroxyle (de préférence méthyle) ;
   q est un entier de 2 à 6 (de préférence 3) ;
   p est 0 ou 1 ;
   r est un nombre entier de 5 à 700 ;
   m est un entier allant de 1 à 3 (de préférence 1) ;
   On utilise plus particulièrement les macromères polysiloxanes de formule : 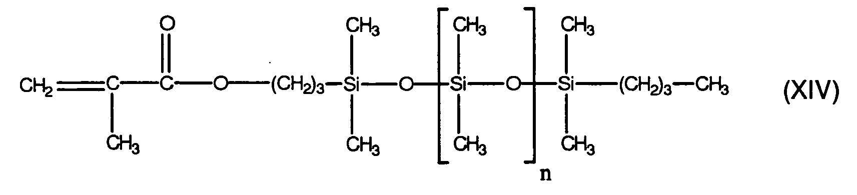 n étant un entier allant de 5 à 700.
   Un mode particulier de réalisation de l'invention consiste à utiliser un copolymère susceptible d'être obtenu par polymérisation radicalaire à partir du mélange de monomères constitué par :
   60 % en poids d'acrylate de tertiobutyle :
   20 % en poids d'acide acrylique ;
   20 % en poids de macromère siliconé de formule : 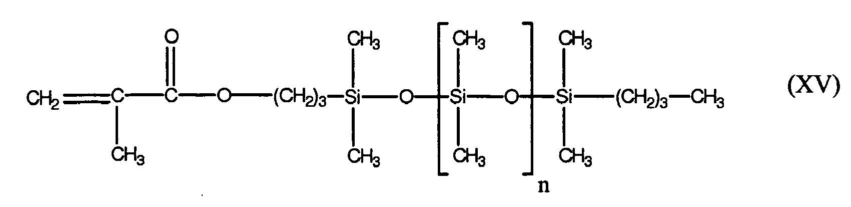 n étant un entier allant de 5 à 700 ; les pourcentages en poids étant calculés par rapport au poids total des monomères.
   Une autre famille particulière de polymères siliconés convenant pour la réalisation de la présente invention est constituée par les copolymères greffés siliconés susceptibles d'être obtenus par extrusion réactive d'un macromère polysiloxane ayant une fonction réactive terminale sur un polymère du type polyoléfine comportant des groupes réactifs susceptibles de réagir avec la fonction terminale du macromère polysiloxane pour former une liaison covalente permettant le greffage de la silicone sur la chaîne principale de la polyoléfine.
   Ces polymères sont décrits ainsi que leur procédé de préparation dans la demande de brevet WO 95/00578.
   Les polyoléfines réactives sont choisies de préférence parmi les polyéthylènes ou les polymères de monomères dérivés de l'éthylène tels que le propylène, le styrène, les alkylstyrènes, le butylène, le butadiène, les (méth)acrylates, les esters de vinyle ou équivalents, comportant des fonctions réactives susceptibles de réagir avec la fonction terminale du macromère polysiloxane. Ils sont choisis plus particulièrement parmi les copolymères d'éthylène ou de dérivés d'éthylène et de monomères choisis parmi ceux comportant une fonction carboxylique tels que l'acide (méth)acrylique ; ceux comportant une fonction anhydride d'acide tels que l'anhydride de l'acide maléique ; ceux comportant une fonction chlorure d'acide tels que le chlorure de l'acide (méth)acrylique ; ceux comportant une fonction ester tels que les esters de l'acide (méth)acrylique ; ceux comportant une fonction isocyanate.
   Les macromères siliconés sont choisis de préférence parmi les polysiloxanes comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires et plus particulièrement parmi ceux répondant à la formule générale (XVI) :

   T-(CH2)s-Si-[(OSiR5R6)t-R7]y (XVI)

   dans laquelle T est choisi dans le groupe constitué par NH2, NHR', une fonction époxy, OH, SH ; R5, R6, R7 et R', indépendamment, désignent un alkyle en C1-C6, phényle, benzyle, ou alkylphényle en en C6-C12, hydrogène ; s est un nombre de 2 à 100 ; t est un nombre de 0 à 1000 et y est un nombre de 1 à 3. Ils ont un poids moléculaire moyen en nombre de préférence allant de 5 000 à 300 000, plus préférentiellement de 8 000 à 200 000 et plus particulièrement de 9 000 à 40 000.
   Selon la présente invention, le ou les polymères siliconés greffés, à squelette polysiloxanique greffé par des monomères organiques non-siliconés comprennent une chaîne principale de silicone (ou polysiloxane (≡Si-O-)n) sur laquelle se trouve greffé, à l'intérieur de ladite chaîne ainsi qu'éventuellement à l'une au moins de ses extrémités, au moins un groupement organique ne comportant pas de silicone.
   Les polymères à squelette polysiloxanique greffé par des monomères organiques non-siliconés selon l'invention peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH2=CH-, ou encore la réaction entre des groupements thio-fonctionnels-SH avec ces mêmes groupements vinyliques.
   Des exemples de polymères à squelette polysiloxanique greffé par des monomères organiques non-siliconés convenant à la mise en oeuvre de la présente invention, ainsi que leur mode particulier de préparation, sont notamment décrits dans les demandes de brevets EP-A-0582152, WO 93/23009 et WO 95/03776 dont les enseignements sont totalement inclus dans la présente description à titre de références non limitatives.
   Selon un mode particulièrement préféré de réalisation de la présente invention, le polymère siliconé, à squelette polysiloxanique greffé par des monomères organiques non-siliconés, mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.
   Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous forme d'un sel.
   Selon la présente invention, les monomères hydrophobes à insaturation éthylénique sont de préférence choisis, seuls ou en mélanges, parmi les esters d'acide acrylique alcanols et/ou les esters d'acide méthacrylique d'alcanols. Les alcanols sont de préférence en C1-C18 et plus particulièrement en C1-C12. Les monomères préférentiels sont choisis dans le groupe constitué par le (méth)acrylate d'isooctyle, le (méth)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.
   Une famille de polymères siliconés à squelette polysiloxanique greffé par des monomères organiques non-siliconés convenant particulièrement bien à la mise en oeuvre de la présente invention est constituée par les polymères siliconés comportant dans leur structure le motif de formule (XVII) suivant : 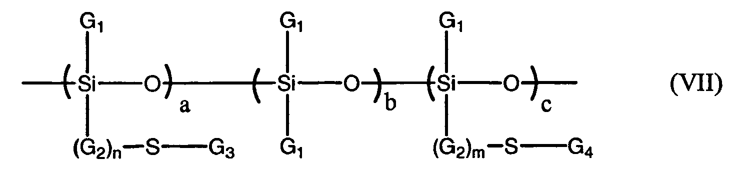 dans lequel les radicaux G1, identiques ou différents, représentent l'hydrogène ou un radical alkyle en C1-C10 ou encore un radical phényle ; les radicaux G2, identiques ou différents, représentent un groupe alkyle en C1-C10 ; G3 représente un reste polymérique résultant de l'(homo)polymérisation d'au moins un monomère anionique à insaturation éthylénique ; G4 représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.
   De préférence, le motif de formule (XVII) ci-dessus présente au moins l'une, et encore plus préférentiellement l'ensemble, des caractéristiques suivantes :
   les radicaux G1 désignent un radical alkyle, de préférence le radical méthyle ;
   n est non nul, et les radicaux G2 représentent un radical divalent en C1-C3, de préférence un radical propylène ;
   G3 représente un radical polymérique résultant de l'(homo)polymérisation d'au moins un monomère du type acide carboxylique à insaturation éthylénique, de préférence l'acide acrylique et/ou l'acide méthacrylique ;
   G4 représente un radical polymérique résultant de l'(homo)polymérisation d'au moins un monomère du type (méth)acrylate d'alkyle(C1-C10), de préférence le (méth)acrylate d'isobutyle ou de méthyle.
   Des exemples de polymères siliconés greffés répondant à la formule (XVII) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.
   De préférence, la masse moléculaire en nombre des polymères siliconés à squelette polysiloxanique greffé par des monomères organiques non-siliconés de l'invention varie de 10 000 à 1 000 000 environ, et encore plus préférentiellement de 10 000 à 100 000 environ.
   A titre de polymères siliconés greffés utilisables selon la présente invention, on peut citer le produit commercialisé par la société 3M sous la référence VS80.
H) Les polyuréthanes anioniques.
   Les polyuréthanes utilisés de préférence selon l'invention présentent de préférence un motif répétitif de base répondant à la formule (XVIII) suivante :

   -X'-B"-X'-CO-NH-R-NH-CO- (XVIII)

   dans laquelle
   X' représente O et/ou NH,
   B" est un radical hydrocarboné divalent, ce radical étant substitué ou non, et
   R est un radical divalent choisi parmi les radicaux alkylènes ramifiés ou non de type aromatique en C6-C20, aliphatique en C1 à C20, de préférence en C1-C6, cycloaliphatique en C1 à C2, de préférence en C1-C6, ces radicaux étant non substitués ou substitués par un ou plusieurs groupements halogène, alcoxy en C1-C4, aryle en C6-C30 en particulier phényle.

De préférence, le radical B" est un radical divalent en C1-C30, de préférence C2-C10 et est porteur d'un groupement présentant une ou des fonctions carboxyliques et/ou une ou des fonctions sulfoniques, lesdites fonctions carboxyliques et/ou sulfoniques étant sous forme libre ou partiellement ou totalement neutralisées par une base minérale ou organique telle que les hydroxydes des métaux alcalins ou alcalinoterreux, l'ammoniaque et les alkylamines ou alcanolamines, les acides aminés organiques. De préférence B" est le radical divalent issu de l'acide diméthylolpropionique.

Le radical R est avantageusement choisi parmi les radicaux répondant aux formules suivantes : 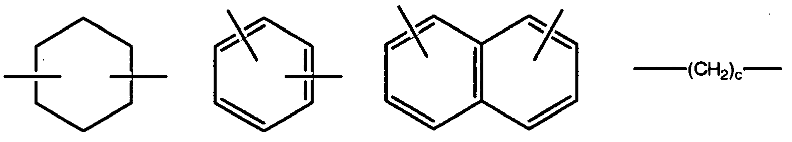 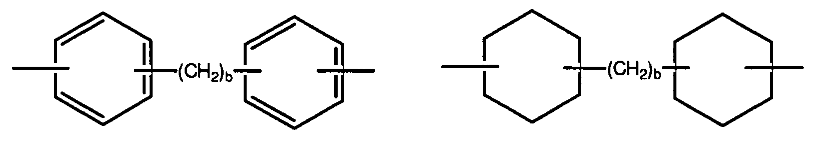 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4 et/ou 2,6-tolylène, 1,5-naphtylène, p-phénylène, méthylène-4,4bis-cyclohexyle et le radical divalent dérivé de l'isophorone.

Les polyuréthanes fixant utilisables peuvent comporter des greffons silicones et des silicones à greffons hydrocarbonés.

Un polyuréthane utilisable peut avantageusement comprendre en outre au moins une séquence polysiloxane et son motif répétitif de base répond par exemple à la formule générale (XIX) :

-X'-P-X'-CO-NH-R-NH-CO- (XIX)

dans laquelle :
P est un segment polysiloxanique,
X' représente O et/ou NH, et
R est un radical divalent choisi parmi les radicaux alkylènes ramifiés ou non de type aromatique en C6-C20, aliphatique en C1-C20, de préférence en C1-C6, cycloaliphatique en C1-C20, de préférence en C1-C6, ces radicaux étant non substitués ou substitués par un ou plusieurs groupements halogène, alcoxy en C1-C4, aryle en C1-C30, en particulier phényle.

Le radical R est avantageusement choisi parmi les radicaux répondant aux formules suivantes : 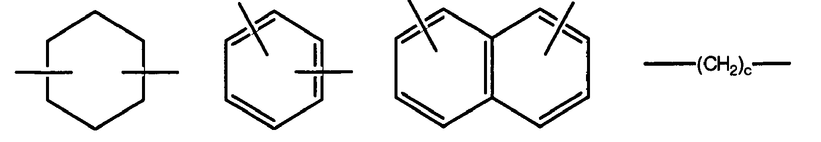 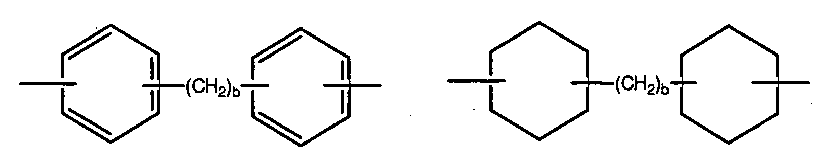 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4-et/ou 2,6-tolylène, 1,5-naphtylène, p-phénylène, méthylène-4,4bis-cyclohexyle et le radical divalent dérivé de l'isophorone.

Avantageusement, le segment polysiloxanique P répond à la formule générale (XX) ci-après : 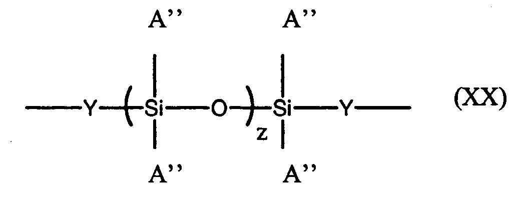 dans laquelle :
les groupes A", qui peuvent être identiques ou différents, sont choisis parmi d'une part les groupes hydrocarbonés monovalents en C1 à C20, substantiellement exempts d'insaturation éthylénique et, d'autre part, les groupes aromatiques,
Y représente un groupe hydrocarboné divalent, et
Z représente un nombre entier, choisi de telle sorte que la masse moléculaire moyenne du segment polysiloxane soit comprise entre 300 et 10 000.

En général, le groupe divalent Y est choisi parmi les groupes alkylène de formule -(CH2)a-, dans laquelle a représente un nombre entier pouvant être compris entre 1 et 10.

Les groupes A" peuvent être choisis parmi les groupes alkyle en C1-C18, en particulier les groupes méthyle, éthyle, propyle, isopropyle, butyle, pentyle, hexyle, octyle, décyle, dodécyle et octadécyle ; les groupes cycloalkyle, en particulier le groupe cyclohexyle ; les groupes aryle, notamment phényle et naphtyle : les groupes arylalkyle, notamment benzyle et phényléthyle, ainsi que les groupes tolyle et xylyle.

A titre d'exemples de polyuréthane fixant, on peut notamment citer le copolymère acide diméthylolpropionique/isophorone-diisocyanate/néopentylglycol/polyesterdiols (connu aussi sous le nom de polyuréthane-1, appellation INCI) vendu sous la marque Luviset® PUR par la société BASF, le copolymère acide diméthylol-propionique/isophorone-diisocyanate/néopentylglycol/polyesterdiols/diamine siliconée (connu aussi sous le nom de polyuréthane-6, appellation INCl) vendu sous la marque Luviset® Si PUR A par la société BASF.

Comme autre polyuréthane anionique, on peut aussi utiliser l'AVALURE UR 450.

On peut également utiliser les polymères à groupements sulfoisophtalates, tels que les polymères AQ55 et AQ48 commercialisés par la société EASTMAN.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG® par la société BASF, les copolymères acide méthacrylique/acrylate d'éthyle, notamment en dispersion aqueuse, tels que les LUVIFLEX SOFT et LUVIMER MAE commercialisés par la société BASF. Des copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléïque monoestérifié vendu sous la dénomination GANTREZ® ES 425 par la société ISP, le LUVISET Sl PUR, le MEXOMERE PW, les polyuréthanes anioniques élastomères ou non, les polymères à groupements sulfoisophtalates, les polymères siliconés greffés anioniques, ainsi que l'AMERHOLD DR 25, le VS 80.

Les polymères amphotères utilisables conformément à l'invention peuvent être choisis parmi les polymères comportant des motifs B"' et C"' répartis statistiquement dans la chaîne polymère où B"' désigne un motif dérivant d'un monomère comportant au moins un atome d'azote basique et C"' désigne un motif dérivant d'un monomère acide comportant un ou plusieurs groupements carboxyliques ou sulfoniques ou bien B"' et C"' peuvent désigner des groupements dérivant de monomères zwittérioniques de carboxybétaïnes ou de sulfobétaïnes ; B"' et C"' peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B"' et C"' font partie d'une chaîne d'un polymère à motif éthylène-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus que l'on préfère plus particulièrement, sont choisis parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkyl-méthacrylamide et acrylamide. De tels composés sont décrits dans le brevet américain n ° 3 836 537.
   Le composé vinylique peut être également un sel de dialkyldiallylammonium tel que le chlorure de diéthyldiallyl-ammonium.
(2) les polymères comportant des motifs dérivant :
   a) d'au moins un monomère choisi parmi les acrylamides ou les méthacrylamides substitués sur l'azote par un groupe alkyle,
   b) d'au moins un comonomère acide contenant un ou plusieurs groupements carboxyliques réactifs, et
      d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.
   Les acrylamides ou méthacrylamides N-substitués plus particulièrement préférés selon l'invention sont les groupements dont les groupes alkyle contiennent de 2 à 12 atomes de carbone et plus particulièrement le N-éthylacrylamide, le N-tertiobutylacrylamide, le N-tertiooctylacrylamide, le N-octylacrylamide. le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides
   ou des anhydrides maléique ou fumarique. Les comonomères basiques préférés sont des méthacrylates d'aminoéthyle, de butylaminoéthyle, de N,N'-diméthylaminoéthyle, de N-tertio-butylaminoéthyle. On utilise particulièrement les copolymères dont la dénomination CTFA (4ème Ed, 1991) est Octylacrylamide/ acrylates/butylaminoethylmethacrylate copolymer tels que les produits vendus sous la dénomination AMPHOMER® ou LOVOCRYL® 47 par la société NATIONAL STARCH.
(3) les polyaminoamides réticulés et alkylés partiellement ou totalement dérivant de polyaminoamides de formule générale : 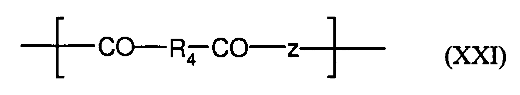 dans laquelle R4 représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis primaire ou bis dérivé secondaire, et Z désigne un groupe d'une polyalkylène-polyamine bis-primaire, mono ou bis-secondaire et représente de préférence :
   a) dans les proportions de 60 à 100 % en moles, le groupe 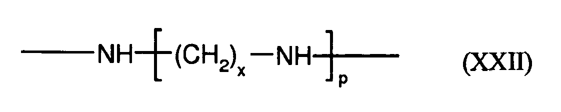 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (XXII) ci-dessus, dans lequel x=2 et p=1 et qui dérive de l'éthylènediamine, ou le groupe dérivant de la pipérazine 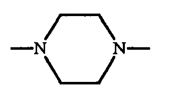
   c) dans les proportions de 0 à 20 % en moles, le groupe
      - NH-(CH2)6-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane-sultone ou de leurs sels.
         Les acides carboxyliques saturés sont choisis de préférence parmi les acides ayant 6 à 10 atomes de carbone tels que les acides adipique, 2,2,4-triméthyladipique et 2,4,4-triméthyladipique, téréphtalique, les acides à double liaison éthylénique comme, par exemple, les acides acrylique, méthacrylique, itaconique. Les alcane-sultones utilisées dans l'alcoylation sont de préférence la propane- ou la butane-sultone, les sels des agents d'alcoylation sont de préférence les sels de sodium ou de potassium.
(4) les polymères comportant des motifs zwittérioniques de formule : 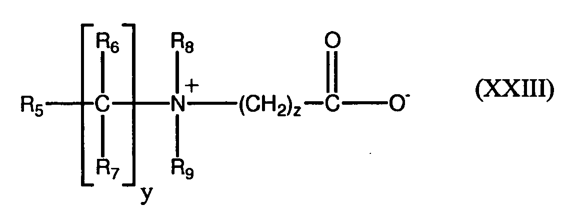 dans laquelle R5 désigne un groupement insaturé polymérisable tel qu'un groupement acrylate, méthacrylate, acrylamide ou méthacrylamide, y et z représentent chacun un nombre entier de 1 à 3, R6 et R7 représentent un atome d'hydrogène, un groupement méthyle, éthyle ou propyle, R8 et R9 représentent un atome d'hydrogène ou un groupe alkyle de telle façon que la somme des atomes de carbone dans R10 et R11 ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle, ou des acrylates ou méthacrylates d'alkyle, des acrylamides ou méthacrylamides, ou l'acétate de vinyle.
(5) les polymères dérivés du chitosane comportant des motifs monomères répondant aux formules suivantes: 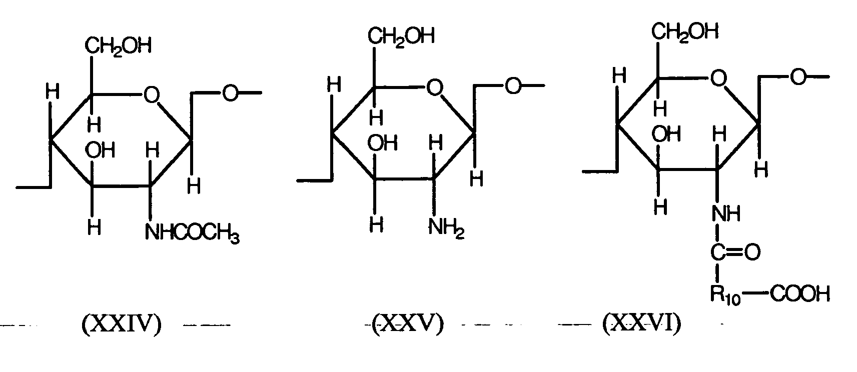 le motif (XXIV) étant présent dans des proportions comprises entre 0 et 30 %, le motif (XXV) dans des proportions comprises entre 5 et 50 % et le motif (XXVI) dans des proportions comprises entre 30 et 90 %, étant entendu que dans ce motif F, R10 représente un groupe de formule: 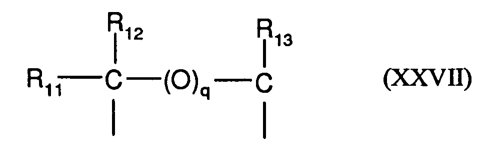 dans laquelle si q=0, R11, R12 et R13, identiques ou différents, représentent chacun un atome d'hydrogène, un reste méthyle, hydroxyle, acétoxy ou amino, un reste monoalkylamine ou un reste dialkylamine éventuellement interrompus par un ou plusieurs atomes d'azote et/ou éventuellement substitués par un ou plusieurs groupes amine, hydroxyle, carboxyle, alkylthio, sulfonique, un reste alkylthio dont le groupe alkyle porte un reste amino, l'un au moins des groupes R17, R18 et R19 étant dans ce cas un atome d'hydrogène ;
   ou si q=1, R11, R12 et R13 représentent chacun un atome d'hydrogène, ainsi que les sels formés par ces composés avec des bases ou des acides.
(6) Les polymères dérivés de la N-carboxyalkylation du chitosane.
(7) Les polymères de motifs répondant à la formule générale (XXVIII) décrits par exemple, dans le brevet français 1 400 366 : 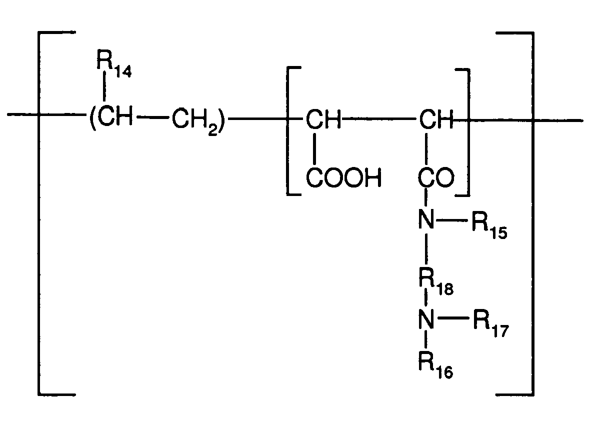 dans laquelle R14 représente un atome d'hydrogène, un groupe CH30, CH3CH2O, phényle, R15 désigne l'hydrogène ou un groupe alkyle inférieur tel que méthyle, éthyle, R16 désigne l'hydrogène ou un groupe alkyle inférieur tel que méthyle, éthyle, R17 désigne un groupe alkyle inférieur tel que méthyle, éthyle ou un groupe répondant à la formule: -R18-N(R16)2, R18 représentant un groupement -CH2-CH2-, -CH2-CH2-CH2-, -CH2-CH(CH3)-, R16 ayant les significations mentionnées ci-dessus,
   ainsi que les homologues supérieurs de ces groupes et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule:

      -D-X"-D-X"-D- (XXIX)

      où D désigne un groupe 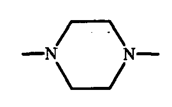 et X" désigne le symbole E ou E', E ou E', identiques ou différents, désignent un groupe bivalent qui est un groupe alkylène à chaîne droite ou ramifiée comportant jusqu'à 7 atomes de carbone dans la chaîne principale non substituée ou substituée par des groupements hydroxyle, et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X"-D-X"- (XXX)
   où D désigne un groupe 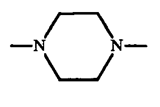 et X" désigne le symbole E ou E' et au moins une fois E'; E ayant la signification indiquée ci-dessus et E' est un groupe bivalent qui est un groupe alkylène à chaîne droite ou ramifiée ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une
   ou plusieurs fonctions hydroxyle et bétaïnisées par réaction avec l'acide chloracétique ou du chloracétate de soude.
(9) Les copolymères alkyl(C1-C5)vinyléther/anhydride maléique modifié partiellement par semiamidification avec une N,N-dialkylaminoalkylamine telle que la N,N-diméthylaminopropyl-amine ou par semiestérification avec une N,N-dialcanolamine Ces copolymères peuvent également comporter d'autres comonomères vinyliques tels que le vinylcaprolactame.

Selon un mode de réalisation préféré de l'invention, les polymères amphotères fixant utilisables dans le procédé selon l'invention peuvent être choisis parmi les copolymères à blocs, ramifiés, comprenant :
(a) des motifs non ioniques dérivés d'au moins un monomère choisi parmi les (méth)acrylates d'alkyle en C1-C20, les N-mono-(alkyle en C2-C12)-(méth)acrylamides et les N,N-di-(alkyle en C2-C12)-(méth)acrylamide,
(b) des motifs anioniques dérivés d'au moins un monomère choisi parmi l'acide acrylique et l'acide méthacrylique, et
(c) des motifs polyfonctionnels dérivés d'au moins un monomère comportant au moins deux groupes fonctionnels insaturés polymérisables,
   et ayant de préférence une structure constituée de blocs hydrophobes sur lesquels sont fixés, par l'intermédiaire des motifs polyfonctionnels (c), plusieurs blocs plus hydrophiles.

De préférence, les polymères amphotères présentent au moins deux températures de transition vitreuse (Tg) dont au moins une est supérieure à 20 °C et l'autre est inférieure à 20°C.

Les polymères amphotères préférés sont les polymères comportant des motifs dérivant :
a) d'au moins un monomère choisi parmi les acrylamides ou les méthacrylamides substitués sur l'azote par un groupe alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

On peut citer en particulier les polymères vendus sous la dénomination AMPHOMER par la société NATIONAL STARCH.

Le milieu de la composition contenant le polymère fixant est un milieu cosmétiquement acceptable aqueux, alcoolique ou hydroalcoolique. Les solvants organiques cosmétiquement acceptables sont de préférence des monoalcools, des polyols ou des éthers de ces alcools ou polyols. On peut citer par exemple l'éthanol, l'isopropanol, le glycérol, le propylèneglycol, l'éther monométhylique de propylèneglycol. L'éthanol est un solvant particulièrement préféré.

Généralement, le ou les polymères fixant représentent de 0,1 à 20 %, de préférence de 1 à 15 %, en poids du poids total de la composition les comprenant. L'application de la composition comprenant un polymère fixant peut se faire par exemple à l'aide d'un spray, c'est à dire un dispositif aérosol ou d'un flacon-pompe dispensant une mousse ou de fines gouttelettes d'une solution.

Selon un mode de réalisation particulier de l'invention, l'étape d'application de la composition réductrice et/ou l'étape de fixation s'effectue(nt) sous chauffage ou est (sont) immédiatement suivie(s) d'un chauffage.

Généralement, le procédé selon l'invention comprend, après l'étape d'application d'une composition réductrice, et/ou après l'étape de fixation par oxydation, une étape de rinçage des fibres kératiniques à l'eau.

La composition réductrice peut également être appliquée au fur et à mesure de l'enroulage des cheveux. Généralement, on laisse ensuite agir la composition réductrice pendant un temps de 1 à 50 minutes, de préférence pendant 1 à 30 minutes.

On peut également, après application de la composition réductrice, soumettre la chevelure à un traitement thermique par chauffage à une température comprise entre 30 et 250°C pendant tout ou partie du temps de pose. Dans la pratique, cette opération peut être conduite au moyen d'un casque de coiffure, d'un sèche-cheveux, d'un fer rond ou d'un fer plat, d'un dispensateur de rayons infrarouges et d'autres appareils chauffants, et dans certains cas sous film plastique.

On réalise ensuite éventuellement un rinçage à l'eau des fibres kératiniques, puis un séchage.

L'étape de séchage peut être complète ou partielle, elle peut être réalisée par exemple avec un sèche-cheveux domestique ou encore un casque ou un bonnet chauffant, ou encore être effectuée grâce à un essorage soigneux. De préférence on utilise un système chauffant. De préférence, le séchage est complet.

On applique ensuite, sur les cheveux secs ou essorés, la composition oxydante permettant de reformer les liaisons disulfures de la kératine, généralement pendant un temps de pose de 2 à 30 minutes. On rince ensuite généralement abondamment la chevelure, de préférence à l'eau, puis on enlève les matériels de mise sous tension éventuellement présents.

Comme expliqué précédemment, le procédé objet de l'invention peut comprendre une étape d'application d'une composition de soin contenant un polymère cationique et/ou une silicone de préférence aminée. La composition de soin peut être appliquée à titre d'exemple aux stades suivants du procédé objet de l'invention :
- avant la mise sous tension des cheveux,
- entre l'étape de mise sous tension des cheveux et l'étape d'application de la composition réductrice,
- après l'étape de rinçage à l'eau suivant l'étape d'application de la composition réductrice et avant l'étape de fixation par oxydation, et/ou
- après l'étape de rinçage à l'eau suivant l'étape de fixation par oxydation.

De préférence, la composition de soin est appliquée avant l'étape de mise sous tension mécanique des fibres kératiniques.

De préférence, l'étape d'application d'une composition de soin est suivie d'un rinçage, de préférence d'un rinçage à l'eau.

Après rinçage du réducteur, les cheveux sont séchés en forme ou essorés, puis fixés avec le fixateur.

Le procédé de déformation permanente selon l'invention, notamment grâce à la présence d'une simple étape intermédiaire de séchage entre l'étape de réduction et l'étape d'oxydation, permet d'apporter durablement, sur plusieurs semaines, de la texture, de la masse, du corps, de la coiffabilité, et du volume en racines aux cheveux, et en particulier aux cheveux fins et courts.

La couleur des cheveux est peu modifiée juste après le traitement.

De plus, un effet protecteur de la couleur artificielle des cheveux est observé après plusieurs lavages par shampoings. En d'autres termes, le procédé de déformation permanente des fibres kératiniques objet de l'invention dégrade peu ou ne dégrade pas la coloration artificielle des cheveux.

Enfin, la présence dans le procédé selon l'invention d'une étape d'application d'une composition réductrice et d'une composition oxydante, permet d'obtenir une déformation durable des fibres kératiniques équivalente à celle observée avec les procédés de déformation permanente classiques.

Un autre objet de l'invention est un kit comprenant :
- au moins un premier compartiment renfermant une composition réductrice telle que décrite ci-dessus, et
- au moins un second compartiment renfermant une composition oxydante, telle que décrite ci-dessus.

De préférence, ce kit comprend également des moyens de mise sous tension mécanique des cheveux, et en particulier des bigoudis tulipe.

L'invention est illustrée par les exemples non limitatifs suivants.

### Exemple 1

On prépare une composition réductrice et une composition oxydante pour la mise en oeuvre d'un procédé de déformation permanente des cheveux selon l'invention.

Les formulations sont les suivantes :

### Exemple de composition

Dans les exemples de composition suivants les pourcentages sont exprimés en poids par rapport au poids total de la composition.

### Composition réductrice (1) :

| | |
|---|---|
| Cystéine | 2% |
| Thioglycolate d'ammonium en | |
| solution aqueuse à 71 % | 0,7% |
| Monoéthanolamine | 1,4% |
| Bicarbonate d'ammonium | 1,3% |
| Cocoamphopropionate de sodium | 0,7% |
| Mexomère PO à 60% de matière active | 0,8% |
| Eau déminéralisée | qsp 100% |

pH 8.8

### Composition oxydante (2)

| | |
|---|---|
| Peroxyde d'hydrogène à 50% | 2,4% |
| Stabilisants | 0,01 % |
| Acide citrique | 0,05% |
| Merquat 100 (Polyquaternium 6) | 0,6% |
| Oxyde de lauramine | 1,1% |
| Eau déminéralisée | qsp 100% |

pH 3

### Composition de spray (3)

| | |
|---|---|
| LUVISET Si PUR A | 5,13% |
| AQ 48 | 6,15% |
| Alcool | 28,95% |
| Eau | qsp 100% |

Aérosol contenant 65g de composition (3) et 35g de propulseur diméthyléther

### Composition de soin 4

| | |
|---|---|
| Wacker Belsil ADM LOG 1 | 10% |
| Eau | qsp 100% |

### Exemple 2 procédés de déformation permanente selon l'invention

### Protocole avec spray 3

La chevelure est shampooinée et essorée.
On procède à l'enroulage de la chevelure avec des bigoudis tulipes.
On applique généreusement la composition (3) en l'aérosol sur l'ensemble de la tête.
On applique ensuite la composition réductrice (1) sur la chevelure. Une pause de 15 minutes est observée, suivie d'un rinçage.
La chevelure est ensuite séchée sous casque chauffant.
La composition oxydante (2) est ensuite appliquée.
Après 5 minutes, la chevelure est rincée et les bigoudis tulipes sont enlevés.
On observe un décollement en racine et une perception de cheveux plus denses. Après plusieurs shampoings, ces avantages sont toujours présents.

### Protocole avec soin 4

La chevelure est shampooinée et essorée.
On applique la composition de soin (4).
On procède à l'enroulage de la chevelure avec des bigoudis tulipes.
On applique ensuite la composition réductrice (1) sur la chevelure.
Une pause de 15 minutes est observée, suivie d'un rinçage.
La chevelure est ensuite séchée au moyen d'un sèche-cheveux.
La composition oxydante (2) est ensuite appliquée.
Après 5 minutes, la chevelure est rincée et les bigoudis tulipes sont enlevés. On observe un décollement en racine et perception de cheveux plus denses. Après plusieurs shampoings, ces avantages sont toujours présents.

## Revendications

1. Procédé de déformation permanente des fibres kératiniques, en particulier des cheveux, comprenant :
- une étape de mise sous tension des fibres kératiniques, puis
- une étape d'application sur les fibres kératiniques d'une composition comprenant un polymère fixant,
- une étape d'application sur les fibres kératiniques d'une composition réductrice, pour réduire les liaisons disulfure de la kératine, ladite composition réductrice comprenant dans un milieu cosmétiquement acceptable, de 0,1 à 3% en poids d'au moins un agent réducteur par rapport au poids total de la composition réductrice, puis après un éventuel rinçage,
- une étape de séchage des fibres kératiniques, puis
- une étape de fixation par oxydation, pour reformer lesdites liaisons, par application sur les fibres kératiniques d'une composition oxydante,
- une étape d'enlèvement des matériels de mise sous tension.

2. Procédé selon la revendication 1 **caractérisé en ce que** le séchage des fibres kératiniques est un séchage partiel ou un séchage complet.

3. Procédé selon la revendication 1 **caractérisé en ce que** le séchage des fibres kératiniques est un séchage complet.

4. Procédé selon l'une quiconque des revendications précédentes, **caractérisé en ce que** l'étape de mise sous tension des fibres kératiniques est réalisée au moyen de bigoudis tulipe.

5. Procédé selon l'une quelconque des revendications précédentes, **caractérisé en ce que** le ou les agents réducteurs sont choisis parmi les sulfites, les bisulfites, les thiols et les phosphines.

6. Procédé selon l'une quelconque des revendications 1 à 4, **caractérisé en ce que** le ou les agents réducteurs sont choisis parmi la L-cystéine, la D-cystéine, la L,D-cystéine, et leurs sels, l'acide thiolactique ses sels et ses esters, l'acide thioglycolique ses sels et ses esters, et leurs mélanges.

7. Procédé selon l'une quelconque des revendications précédentes **caractérisé en ce que** le ou les agents réducteurs représentent de 0,3 à 3%, en poids par rapport au poids total de la composition réductrice.

8. Procédé selon l'une quelconque des revendications précédentes **caractérisé en ce qu'**on laisse la composition réductrice agir pendant 2 à 50 minutes, de préférence pendant 2 à 30 minutes, et mieux encore pendant 5 à 20 minutes.

9. Procédé selon l'une quelconque des revendications précédentes **caractérisé en ce que** le pH de la composition réductrice est compris entre 7,5 et 11, de préférence entre 8 et 9.5.

10. Procédé selon l'une quelconque des revendications précédentes **caractérisé en ce que** la composition oxydante comprend au moins un agent oxydant choisi parmi le peroxyde d'hydrogène, le peroxyde d'urée, les bromates alcalins, les polythionates, les persels, tels que les perborates, les percarbonates et les persulfates.

11. Procédé selon la revendication 10 **caractérisé en ce que** le ou les agents oxydants représentent généralement de 0,1 à 10%, de préférence de 0,5 à 5%, en poids par rapport au poids total de la composition oxydante.

12. Procédé l'une quelconque des revendications précédentes **caractérisé en ce qu'**on laisse la composition oxydante agir pendant 2 à 30 minutes, de préférence pendant 2 à 15 minutes, mieux de 2 à 7 minutes.

13. Procédé selon l'une quelconque des revendications précédentes **caractérisé en ce que** le pH de la composition oxydante varie de 1,5 à 4,5, de préférence de 2 à 3,5.

14. Procédé selon l'une quelconque des revendications précédentes **caractérisé en ce qu'**il comprend, après l'étape d'application d'une composition réductrice, et/ou après l'étape de fixation par oxydation, une étape de rinçage des fibres kératiniques à l'eau.

15. Procédé selon l'une quelconque des revendications précédentes **caractérisé en ce que** l'étape d'application de la composition réductrice s'effectue sous chauffage ou est immédiatement suivie d'un chauffage.

16. Procédé selon l'une quelconque des revendications précédentes **caractérisé en ce que** la composition réductrice et la composition oxydante se présentent indépendamment l'une de l'autre sous la forme de lotion, de gel épaissi ou non, de mousse, ou de crème.

17. Procédé de déformation permanente selon l'une quelconque des revendications précédentes **caractérisé en ce que** l'étape de mise sous tension mécanique des fibres kératiniques est précédée d'une étape d'application d'une composition de soin contenant un polymère cationique et/ou une silicone, de préférence, une silicone aminée.

18. Procédé de déformation permanente selon la revendication 17 **caractérisé en ce que** l'étape d'application d'une composition de soin est suivie d'un rinçage.

## Claims

1. A method for permanently reshaping the keratin fibers, especially hair keratin fibers, comprising:
- a step for setting the keratin fibers under tension, then
- a step for applying a fixing polymer-containing composition onto the keratin fibers,
- a step for applying a reducing composition onto the keratin fibers so as to reduce the keratin disulfide bonds, said reducing composition comprising in a cosmetically acceptable medium, from 0.1 to 3% by weight of at least one reducing agent, as related to the reducing composition total weight, then, after an optional rinsing operation,
- a step for drying the keratin fibers, then
- an oxidation fixing step, so as to reform said bonds, by applying an oxidizing composition onto the keratin fibers,
- a step of removing the tensioning materials.

2. A method according to claim 1, **characterized in that** the keratin fiber drying is a partial drying or a complete drying.

3. method according to claim 1, **characterized in that** the keratin fiber drying is a complete drying.

4. A method according to any preceding claim, **characterized in that** the keratin fiber tensioning step is carried out by means of tulip-type curlers.

5. A method according to any preceding claim, **characterized in that** the reducing agent(s) is or are selected from the group consisting of sulfites, bisulfites, thiols and phosphines.

6. A method according to any of claims 1 to 4, **characterized in that** the reducing agent(s) is or are selected from the group consisting of L-cysteine, D-cysteine, L,D-cysteine, and their salts, thiolactic acid, salts and esters thereof, thioglycolic acid, salts and esters thereof, and mixtures thereof.

7. A method according to any preceding claim, **characterized in that** the reducing agent(s) represent(s) from 0.3 to 3% by weight, as related to the reducing composition total weight.

8. A method according to any preceding claim, **characterized in that** the reducing composition is allowed to react for 2 to 50 minutes, preferably for 2 to 30 minutes, and even more preferably for 5 to 20 minutes.

9. A method according to any preceding claim, **characterized in that** the pH value of the reducing composition does vary from 7.5 to 11, preferably from 8 to 9.5.

10. A method according to any preceding claim, **characterized in that** the oxidizing composition comprises at least one oxidizing agent selected from hydrogen peroxide, carbamide peroxide, alkaline bromates, polythionates, persalts, such as perborates, percarbonates and persulfates.

11. A method according to claim 10, **characterized in that** the oxidizing agent(s) generally represent(s) from 0.1 to 10%, preferably from 0.5 to 5% by weight as related to the oxidizing composition total weight.

12. A method according to any preceding claim, **characterized in that** the oxidizing composition is allowed to react for 2 to 30 minutes, preferably for 2 to 15 minutes, more preferably for 2 to 7 minutes.

13. A method according to any preceding claim, **characterized in that** the pH value of the oxidizing composition does vary from 1.5 to 4.5, preferably from 2 to 3.5.

14. A method according to any preceding claim, **characterized in that** it comprises, following the reducing composition applying step and/or following the oxidation fixing step, a step consisting in rinsing the keratin fibers with water.

15. A method according to any preceding claim, **characterized in that** the reducing composition applying step is effected under heating or is immediately followed with a heating operation.

16. A method according to any preceding claim, **characterized in that** the reducing composition and the oxidizing composition present independently from each other in the form of a lotion, a gel, thickened or not, a foam, or a cream.

17. A method for permanently reshaping the hair according to any preceding claim, **characterized in that** the step for mechanically setting the keratin fibers under tension does occur after a hair-care composition applying step which contains a cationic polymer and/or a silicone, preferably, an amino silicone.

18. A method for permanently reshaping the hair according to claim 17, **characterized in that** the hair-care composition applying step is followed with a rinsing operation.

## Patentansprüche

1. Verfahren zur permanenten Verformung von Keratinfasern, insbesondere von Haaren, umfassend:
- eine Stufe des Unter-Spannung-Bringens der Keratinfasern, danach
- eine Stufe der Auftragung einer Zusammensetzung, die ein fixierendes Polymer umfasst, auf die Keratinfasern,
- eine Stufe der Auftragung einer reduzierenden Zusammensetzung auf die Keratinfasern, um die Disulfidbindungen des Keratins zu reduzieren, wobei der reduzierende Zusammensetzung in einem kosmetisch verträglichen Medium 0,1 bis 3 Gew.-%, bezogen auf das Gesamtgewicht der reduzierenden Zusammensetzung, wenigstens eines Reduktionsmittels umfasst, danach ein potenzielles Ausspülen,
- eine Stufe der Trocknung der Keratinfasern, danach
- eine Stufe der Fixierung durch Oxydation, um die Bindungen wiederherzustellen, durch Auftragung einer oxidierenden Zusammensetzung auf die Keratinfasern,
- eine Stufe der Entfernung der Materialien zum Unter-Spannung-Bringen.

2. Verfahren gemäß Anspruch 1, **dadurch gekennzeichnet, dass** die Trocknung der Keratinfasern eine partielle Trocknung oder eine vollständige Trocknung ist.

3. Verfahren gemäß Anspruch 1, **dadurch gekennzeichnet, dass** die Trocknung der Keratinfasern eine vollständige Trocknung ist.

4. Verfahren gemäß einem der vorangehenden Ansprüche, **dadurch gekennzeichnet, dass** die Stufe des Unter-Spannung-Bringens der Keratinfasern mit Hilft vorn tulpenförmigen Lockenwicklern (bigoudis tulipe) durchgeführt wird.

5. Verfahren gemäß einem der vorangehenden Ansprüche, **dadurch gekennzeichnet, dass** das Reduktionsmittel oder die Reduktionsmittel aus Sulfiten, Bisulfiten, Thiolen und Phosphinen ausgewählt ist/sind.

6. Verfahren gemäß einem der Ansprüche 1 bis 4, **dadurch gekennzeichnet, dass** das Reduktionsmittel oder die Reduktionsmittel aus L-Cystein, D-Cystein, L,D-Cystein und ihren Salzen, Thiomilchsäure, ihren Salzen und ihren Estern, Thioglykolsäure, ihren Salzen und ihren Estern und deren Gemischen ausgewählt ist/sind.

7. Verfahren gemäß einem der vorangehenden Ansprüche, **dadurch gekennzeichnet, dass** das Reduktionsmittel oder die Reduktionsmittel 0,3 bits 3%, bezogen auf das Gesamtgewicht der reduzierenden Zusammensetzung, darstellen.

8. Verfahren gemäß einem der vorangehenden Ansprüche, **dadurch gekennzeichnet, dass** man die reduzierend Zusammensetzung während 2 bis 50 Minuten, vorzugweise während 2 bis 30 Minuten und noch besser während 5 bis 20 Minuten einwirken lässt.

9. Verfahren gemäß einem der vorangehenden Ansprüche, **dadurch gekennzeichnet, dass** der pH der reduzierenden Zusammensetzung zwischen 7,5 und 11, vorzugsweise zwischen 8 und 9,5 liegt.

10. Verfahren gemäß einem der vorangehenden Ansprüche, **dadurch gekennzeichnet, dass** die oxidierende Zuaammensetzung wenigstens ein Oxidationsmittel umfaßt, das aus Wasserstoffperoxid, Harnstoffperoxid, Alkalibromaten, Polythionaten, Persalzen wie zum Beispiel Perborate, Percarbonate und Persulfate, ausgewählt wird.

11. Verfahren gemäß Anspruch 10, **dadurch gekennzeichnet, dass** das Oxidationsmittel oder die Oxidationsmittel im Allegemeinen 0,1 bis 10 Gew.-%, vorzugsweise 0,5 bis 5 Gew.-%, bezogen auf das Gesamtgewicht der oxidierenden Zusammensetzung, darstellen.

12. Verfahren gemäß einem der vorangehenden Ansprüche, **dadurch gekennzeichnet, dass** man die oxidierende Zusammensetzung während 2 bis 30 Minuten, vorzugsweise 2 bis 15 Minuten, besser 2 bis 7 Minuten einwirken lässt.

13. Verfahren gemäß einem der vorangehende Ansprüche, **dadurch gekennzeichnet, dass** der pH der oxidierenden Zusammensetzung von 1,5 bis 4,5, vorzugweise von 2 bis 3,5 variiert.

14. herfahren gemäß einem der vorangehenden Ansprüche, **dadurch gekennzeichnet, dass** es nach der Stufe der Auftragung einer reduzierenden Zusammensetzung und/oder nach der Stufe der Fixierung durch Oxydation eine Stufe des Ausspülens der Keratinfasern mit Wasser umfaßt.

15. Verfahren gemäß einem der vorangehenden Ansprüche, **dadurch gekennzeichnet, dass** die Stufe der Auftragung der reduzierenden Zusammensetzung unter Erwärmen erfolgt oder ummittelbar darauf ein Erwärmen erfolgt.

16. Verfahren gemäß seinem der vorangehenden Ansprüche, **dadurch gekennzeichnet, dass** die reduzierende Zusammensetzung und die oxidierende Zusammensetzung sich unabhängig voneinander in der Form einer Lotion, eines verdickten oder nicht verdickten Gels, eines Schaums oder einer Creme präsentieren.

17. Verfahren zur permanenten Vorformung gemäß einem der vorangehenden Ansprüche, **dadurch gekennzeichnet, dass** der Stufe des unter mechanischer Spannung Bringens der Keratinfasern eine Stufe der Anwendung einer Pflegezusammensetzung vorausgeht, welche ein kationisches Polymer und/oder ein Silikon, vorzugsweise ein aminiertes Silicon, umfasst.

18. Verfahren zur permanenten Vorformung gemäß Anspruch 17, **dadurch gekennzeichnet, dass** auf die Stufe der Auftragung einer Pflegezusammensetzung ein Ausspülen folgt.
